(19) Europäisches Patentamt
European Patent Office
Office européen des brevets

(11) EP 2 739 136 B1

(12) # EUROPÄISCHE PATENTSCHRIFT

(45) Veröffentlichungstag und Bekanntmachung des Hinweises auf die Patenterteilung:
**31.05.2017 Patentblatt 2017/22**

(21) Anmeldenummer: **12741260.9**

(22) Anmeldetag: **31.07.2012**

(51) Int Cl.:
*A01N 25/00* (2006.01) *C09D 7/00* (2006.01)
*C11D 3/22* (2006.01) *C11D 17/00* (2006.01)
*A61Q 5/02* (2006.01) *A61Q 19/10* (2006.01)
*A61K 8/49* (2006.01)

(86) Internationale Anmeldenummer:
**PCT/EP2012/003245**

(87) Internationale Veröffentlichungsnummer:
**WO 2013/017256 (07.02.2013 Gazette 2013/06)**

(54) **VERWENDUNG VON ISOSORBIDDIESTERN ALS VERDICKER**

USE OF ISOSORBIDE DIESTERS AS THICKENERS

UTILISATION DES DIESTERS D'ISOSORBIDE COMME AGENT EPAISSISSANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.08.2011 DE 102011109428**

(43) Veröffentlichungstag der Anmeldung:
**11.06.2014 Patentblatt 2014/24**

(73) Patentinhaber: **Clariant International Ltd**
**4132 Muttenz (CH)**

(72) Erfinder:
- **PILZ, Maurice, Frederic**
  **60329 Frankfurt am Main (DE)**
- **KLUG, Peter**
  **63762 Großostheim (DE)**
- **SCHERL, Franz-Xaver**
  **84508 Burgkirchen (DE)**

(74) Vertreter: **Holmes, Rosalind et al**
**Clariant Produkte (Deutschland) GmbH**
**Patent & License Management Chemicals**
**Industriepark Höchst, G 860**
**65926 Frankfurt am Main (DE)**

(56) Entgegenhaltungen:
**EP-A1- 2 239 315** **WO-A1-2006/103338**
**WO-A1-2008/155159** **WO-A2-2010/108738**
**WO-A2-2010/136121** **US-A1- 2010 113 664**
**US-A1- 2011 117 036**

- **DATABASE CA [Online] CHEMICAL ABSTRACTS SERVICE, COLUMBUS, OHIO, US; 24. Februar 1985 (1985-02-24), "Cosmetics containing isosorbide fatty acid diesters", XP002684643, gefunden im STN Database accession no. 1985:67233 & JP 59 175408 A (NIHON SURFACTANTS INDUSTRY CO., LTD., JAPAN) 4. Oktober 1984 (1984-10-04)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 739 136 B1

## Beschreibung

Die vorliegende Erfindung betrifft die Verwendung von Isosorbiddiestern als Verdicker.

In der Industrie werden Verdicker verwendet, um die Viskosität von Produkten wie beispielsweise von kosmetischen, dermatologischen oder pharmazeutischen Zusammensetzungen, Pflanzenschutzformulierungen, Wasch- oder Reinigungsmittel oder Farb- oder Anstrichmittel gezielt einzustellen. Es sind zahlreiche Verdicker für diesen Zweck bekannt. Diese Verdicker können synthetisch hergestellt worden sein oder auf natürlichen Rohstoffen basieren.

Nachteilig an der Verwendung vieler synthetisch hergestellter Verdicker, z. B. synthetischer Polymere, ist jedoch, dass ihre Herstellung oftmals aufwändig ist und auf synthetischen Rohstoffen basiert. Nachteilig an der Verwendung vieler auf natürlichen Rohstoffen basierenden Verdickern ist demgegenüber, dass das Erscheinungsbild vieler Produkte, die derartige Verdicker enthalten, verbesserungsbedürftig ist.

Es bestand deshalb die Aufgabe, Verdicker zur Verfügung zu stellen, die die oben genannten Nachteile nicht aufweisen oder diese zumindest teilweise verbessern und zudem eine vorteilhafte Verdickungsleistung zeigen.

Überraschend wurde nun gefunden, dass diese Aufgabe gelöst wird durch Verbindungen der Formel (I)

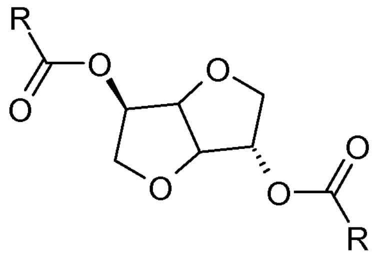

(I)

worin

R eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, wobei die eine oder die mehreren Verbindungen der Formel (I) in einer Zusammensetzung nach Anspruch 1 verwendet wird.

Gegenstand der Erfindung ist somit die Verwendung einer oder mehrerer Verbindungen der Formel (I)

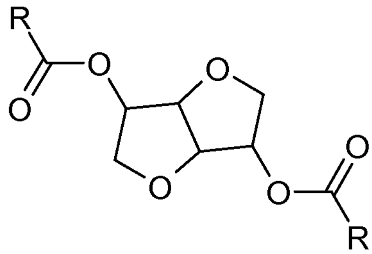

(I)

worin

R eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, wobei die eine oder die mehreren Verbindungen der Formel (I) in einer Zusammensetzung enthaltend eine oder mehrere andere Substanzen ausgewählt aus der Gruppe bestehend aus Sorbitol, Sorbitolestern, Sorbitan, Sorbitanestern, Isosorbid, Isosorbidmonoestern und Carbonsäuren verwendet werden

und die OH-Zahl (als mgKOH/g) der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbidmonoestern und Carbonsäuren in der Zusammensetzung kleiner oder gleich 250 ist,

und wobei die Zusammensetzung eine oder mehrere Verbindungen ausgewählt aus Sorbitol und Sorbitolestern enthält,

und wobei die Zusammensetzung eine oder mehrere Verbindungen der Formel (I) und zusätzlich

II) 0,001 bis 0,2 Gewichtsteile an dem einem oder den mehreren Isosorbidmonoestern der Formel (II)

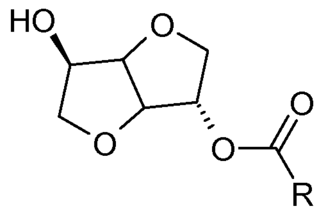

(II)

worin R die oben angegebene Bedeutung besitzt,

enthält, jeweils bezogen auf 1,0 Gewichtsteile an der einen oder den mehreren Verbindungen der Formel (I), als Verdicker.

Die Verbindungen der Formel (I) basieren auf nachwachsenden Rohstoffen wie z. B. auf Isosorbid, welches aus Zucker gewonnen werden kann. Ihre Herstellung ist in einfacher Weise nach dem Fachmann geläufigen Methoden, z. B. durch Veresterung von Isosorbid mit einer Säurekomponente wie einer Carbonsäure, möglich. Das Erscheinungsbild der Produkte, die die Verbindungen der Formel (I) enthalten, wird durch deren Anwesenheit nicht nachteilig beeinflusst. Beispielsweise können im Vergleich zur Verwendung von Xanthan Gum, welches als natürlicher Verdicker oftmals zu Produkten mit einer "brüchigen" bzw. unregelmäßigen oder nicht glatten Oberfläche führt, mit Hilfe der Verbindungen der Formel (I) verdickte Produkte hergestellt werden, die eine glatte Oberfläche aufweisen.

Zusammensetzungen, die zumindest zum Teil auf nachwachsenden Rohstoffen basieren und als Verdicker verwendet werden können, sind bereits bekannt.

WO 2010/108738 A2 (Evonik) beschreibt Formulierungen zur Reinigung und Pflege von menschlichen oder tierischen Körperteilen enthaltend Sorbitancarbonsäureester, wobei sich der Carbonsäureanteil des Sorbitancarbonsäureesters ableitet von einer Carbonsäure enthaltend 6 bis 10 Kohlenstoffatome und die Sorbitancarbonsäureester eine Hydroxylzahl (OH-Zahl) von größer 350 aufweisen sowie die Verwendung der genannten Sorbitancarbonsäureester als Viskositätsregler, Pflegewirkstoff, Schaumbooster oder Solubilisator in reinigenden oder pflegenden Formulierungen. EP2239315 (Cognis) offenbart die Verwendung von Isosorbidmonoestern als Verdickungsmittel in wässrigen Detergenzien und Reinigungsmitteln, macht jedoch keine Aussage über die Hydroxylzahl und schliesst den Einsatz mit Sorbitol und Sorbitolestern aus.

Verbindungen der Formel (I) können z. B. nach dem Fachmann geläufigen Methoden hergestellt werden. Beispielsweise können die Verbindungen der Formel (I) durch Veresterung von Isosorbid nach üblichen und dem Fachmann bekannten Methoden hergestellt werden, wobei sowohl Isosorbid selbst als auch die zur Veresterung verwendeten Säurekomponenten wiederum käuflich erwerbbar sind.

Vorzugsweise ist der Rest R in der einen oder den mehreren Verbindungen der Formel (I) ein linearer gesättigter Alkylrest mit 7 bis 9 Kohlenstoffatomen.

Besonders bevorzugt ist der Rest R in der einen oder den mehreren Verbindungen der Formel (I) ein linearer gesättigter Alkylrest mit 7 Kohlenstoffatomen.

Die eine oder die mehreren Verbindungen der Formel (I) sind in Zusammensetzungen enthaltend eine oder mehrere andere Substanzen erfindungsgemäß als Verdicker verwendet werden. Diese Zusammensetzungen werden im Folgenden als "Zusammensetzungen A" bezeichnet.

Die Zusammensetzungen A enthalten eine oder mehrere Verbindungen der Formel (I) und zusätzlich eine oder mehrere andere Substanzen ausgewählt aus der Gruppe bestehend aus Sorbitol, Sorbitolestern (bei Sorbitolestern kann es sich um Mono-, Di-, Tri-, Tetra-, Penta- und/oder Hexaester handeln), Sorbitan, Sorbitanestern (bei Sorbitanestern kann es sich um Mono-, Di-, Tri- und/oder Tetraester handeln), Isosorbid, Isosorbidmonoestern und Carbonsäuren. Bei "Sorbitan" kann es sich beispielsweise um 1,4- oder 1,5-Sorbitan handeln. Sowohl die Carbonsäuren selbst als auch die den Säurekomponenten der genannten Ester zu Grunde liegenden Carbonsäuren entsprechen der Formel RCOOH, worin R die bei Formel (I) angegebene Bedeutung besitzt und vorzugsweise ein linearer gesättigter Alkylrest mit 7 Kohlenstoffatomen ist, d.h. die Carbonsäure RCOOH vorzugsweise Caprylsäure ist. Die Menge der einen oder der mehreren Verbindungen der Formel (I), bezogen auf die Gesamtmenge der Verbindungen ausgewählt aus der Gruppe bestehend aus der einen oder den mehreren Verbindungen der Formel (I), Sorbitol, Sorbitolestern, Sorbitan, Sorbitanestern, Isosorbid, Isosorbidmonoestern und Carbonsäuren ist vorzugsweise größer oder gleich 70 Gew.-%, besonders bevorzugt größer oder gleich 80 Gew.-% und insbesondere bevorzugt größer oder gleich 85 Gew.-%.

Die Zusammensetzungen A enthalten eine oder mehrere Verbindungen der Formel (I) und zusätzlich

II) 0,001 bis 0,2 Gewichtsteile an dem einem oder den mehreren Isosorbidmonoester der Formel (II)

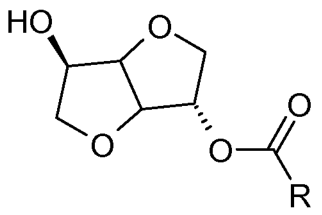

(II)

worin R die oben bei Formel (I) angegebene Bedeutung besitzt, jeweils bezogen auf 1,0 Gewichtsteile an der einen oder den mehreren Verbindungen der Formel (I),
und wobei der Isosorbidmonoester vorzugsweise Isosorbidmonocaprylat ist.

Hierunter wiederum bevorzugt enthalten die soeben genannten Zusammensetzungen A eine oder mehrere Verbindungen der Formel (I) und zusätzlich

II) 0,01 bis 0,15 und besonders bevorzugt 0,05 bis 0,13 Gewichtsteile an dem einen oder den mehreren Isosorbidmonoestern der Formel (II), wobei der Isosorbidmonoester vorzugsweise Isosorbidmonocaprylat ist,

jeweils bezogen auf 1,0 Gewichtsteile an der einen oder den mehreren Verbindungen der Formel (I) und vorzugsweise bezogen auf 1,0 Gewichtsteile an Isosorbidd icaprylat.

In einer hierunter wiederum bevorzugten Ausführungsform der Erfindung enthalten die soeben genannten Zusammensetzungen A entweder keine Carbonsäure RCOOH oder bis zu 0,1, vorzugsweise 0,0001 bis 0,05 und besonders bevorzugt 0,001 bis 0,01 Gewichtsteile Carbonsäure RCOOH, wobei R die oben bei Formel (I) angegebene Bedeutung besitzt, und wobei die Carbonsäure vorzugsweise Caprylsäure ist, bezogen auf 1,0 Gewichtsteile an der einen oder den mehreren Verbindungen der Formel (I) und vorzugsweise bezogen auf 1,0 Gewichtsteile an Isosorbiddicaprylat.

In einer weiteren besonders bevorzugten Ausführungsform der Erfindung enthalten die Zusammensetzungen A einen oder mehrere Sorbitanester aus Sorbitan und Carbonsäuren $R^aCOOH$, vorzugsweise ausgewählt aus Sorbitanestern aus 1,4- und/oder 1,5-Sorbitan und Carbonsäuren $R^aCOOH$, wobei $R^a$ eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, und wobei das Gewichtsverhältnis der einen oder der mehreren Verbindungen der Formel (I) zu dem einen oder den mehreren soeben genannten Sorbitanestern von 70 : 30 bis 100 : 0, vorzugsweise von 80 : 20 bis 100 : 0, besonders bevorzugt von 90 : 10 bis 100 : 0 und insbesondere bevorzugt von 95 : 5 bis 100 : 0 ist. Das angegebene Gewichtsverhältnis von "100 : 0" bedeutet, dass die soeben genannten Zusammensetzungen A in dieser besonders bevorzugten Ausführungsform der Erfindung keinen Sorbitanester enthalten müssen.

Unter den soeben genannten Zusammensetzungen A sind solche bevorzugt, worin der eine oder die mehreren Sorbitanester aus Sorbitan und Carbonsäuren $R^aCOOH$ ausgewählt sind aus Sorbitanestern aus Sorbitan und Caprylsäure und vorzugsweise ausgewählt sind aus Sorbitanestern aus 1,4- und/oder 1,5-Sorbitan und Caprylsäure und der Sorbitanester besonders bevorzugt Sorbitandicaprylat ist.

In diesen Zusammensetzungen A ist die OH-Zahl der Mischung aus der einen oder den mehreren Verbindungen der Formel (I) und der einen oder den mehreren (gegebenenfalls enthaltenen) Sorbitanestern aus Sorbitan und Carbonsäuren $R^aCOOH$ vorzugsweise kleiner oder gleich 250, besonders bevorzugt kleiner oder gleich 200, insbesondere bevorzugt kleiner oder gleich 150 und außerordentlich bevorzugt kleiner oder gleich 100.

In der Erfindung ist die OH-Zahl der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbidmonoestern und Carbonsäuren in den Zusammensetzungen A kleiner oder gleich 250, vorzugsweise kleiner oder gleich 200, besonders bevorzugt kleiner oder gleich 150 und insbesondere bevorzugt kleiner oder gleich 100.

In einer weiteren besonders bevorzugten Ausführungsform der Erfindung enthalten die Zusammensetzungen A keine Verbindungen ausgewählt aus Sorbitol und Sorbitolestern.

In einer weiteren besonders bevorzugten Ausführungsform der Erfindung enthalten die Zusammensetzungen A keine Verbindungen ausgewählt aus Sorbitan und Sorbitanestern.

Die Zusammensetzungen A enthalten eine oder mehrere Verbindungen ausgewählt aus Sorbitol und Sorbitolestern (wobei die der Säurekomponente dieser Ester zu Grunde liegende Carbonsäure vorzugsweise Caprylsäure ist).

Diese Verbindungen sind gemeinsam vorzugsweise in einer Menge kleiner oder gleich 5,0 Gew.-%, besonders

bevorzugt in einer Menge kleiner oder gleich 3,0 Gew.-%, insbesondere bevorzugt in einer Menge kleiner oder gleich 1,0 Gew.-% und außerordentlich bevorzugt in einer Menge kleiner oder gleich 0,5 Gew.-% in den Zusammensetzungen A enthalten, wobei die Angaben in Gew.-% jeweils auf das Gesamtgewicht der fertigen Zusammensetzungen A bezogen sind.

Sofern die Zusammensetzungen A eine oder mehrere Verbindungen ausgewählt aus Sorbitan und Sorbitanestern (wobei die der Säurekomponente dieser Ester zu Grunde liegende Carbonsäure vorzugsweise Caprylsäure ist) enthalten, sind diese Verbindungen gemeinsam vorzugsweise in einer Menge kleiner oder gleich 20,0 Gew.-%, besonders bevorzugt in einer Menge kleiner oder gleich 10,0 Gew.-%, insbesondere bevorzugt in einer Menge kleiner oder gleich 5,0 Gew.-% und außerordentlich bevorzugt in einer Menge kleiner oder gleich 1,0 Gew.-% in den Zusammensetzungen A enthalten, wobei die Angaben in Gew.-% jeweils auf das Gesamtgewicht der fertigen Zusammensetzungen A bezogen sind.

In einer weiteren besonders bevorzugten Ausführungsform der Erfindung enthalten die Zusammensetzungen A die eine oder die mehreren Verbindungen der Formel (I) in Mengen von mindestens 50 Gew.-%, vorzugsweise in Mengen von mindestens 60 Gew.-% und besonders bevorzugt in Mengen von mindestens 70 Gew.-%, jeweils bezogen auf das Gesamtgewicht der fertigen Zusammensetzungen A.

Unter der Hydroxyl- oder OH-Zahl einer Substanz wird diejenige Menge KOH in mg verstanden, die der bei der Acetylierung von 1 g Substanz gebundenen Menge an Essigsäure äquivalent ist.

Geeignete Bestimmungsmethoden zur Ermittlung der OH-Zahl sind z. B. DGF C-V 17 a (53), Ph. Eur. 2.5.3 Method A und DIN 53240.

Im Rahmen der vorliegenden Erfindung werden die OH-Zahlen in Anlehnung an DIN 53240-2 bestimmt. Hierbei wird wie folgt vorgegangen: Es wird 1 g auf 0,1 mg genau von der homogenisierten zu messenden Probe eingewogen. 20,00 ml Acetylierungsgemisch (Acetylierungsgemisch: in 1 Liter Pyridin werden 50 ml Essigsäureanhydrid eingerührt) werden zugeben. Die Probe wird vollständig im Acetylierungsgemisch gelöst, gegebenenfalls unter Rühren und Erwärmen. 5 ml Katalysatorlösung (Katalysatorlösung: 2 g 4-Dimethylaminopyridin werden in 100 ml Pyridin gelöst) werden zugeben. Das Reaktionsgefäß wird verschlossen und 10 Minuten in das auf 55 °C vorgeheizte Wasserbad gestellt und dabei durchmischt. Die Reaktionslösung wird danach mit 10 ml vollentsalztem Wasser versetzt, das Reaktionsgefäß erneut verschlossen und nochmals im Schüttelwasserbad 10 Minuten abreagieren gelassen. Die Probe wird auf Raumtemperatur (25 °C) abgekühlt. Anschließend werden 50 ml 2-Propanol und 2 Tropfen Phenolphthalein zugeben. Diese Lösung wird mit Natronlauge (Natronlauge c = 0,5 mol/l) titriert (Va). Unter den gleichen Bedingungen, jedoch ohne Probeneinwaage, wird der Wirkwert des Acetylierungsgemisches bestimmt (Vb).

Aus dem Verbrauch der Wirkwertbestimmung und der Titration der Probe wird die OH-Zahl (OHZ) nach folgender Formel berechnet:

$$OHZ = \frac{(Vb - Va) \cdot c \cdot t \cdot M}{E}$$

| | |
|---|---|
| OHZ | = Hydroxylzahl in mg KOH/g Substanz |
| Va | = Verbrauch an Natronlauge in ml bei der Titration der Probe |
| Vb | = Verbrauch an Natronlauge in ml bei der Titration des Wirkwertes |
| c | = Stoffmengenkonzentration der Natronlauge in mol/l |
| t | = Titer der Natronlauge |
| M | = Molare Masse von KOH = 56,11 g/mol |
| E | = Probeneinwaage in g |

(Vb - Va) ist diejenige Menge der verwendeten Natronlauge in ml, die der bei der oben beschriebenen Acetylierung der zu messenden Probe gebundenen Menge an Essigsäure äquivalent ist.

Die soeben beschriebene Methode zur Bestimmung der OH-Zahl wird im Folgenden als "Methode OHZ-A" bezeichnet.

Vorzugsweise findet die erfindungsgemäße Verwendung in kosmetischen, dermatologischen oder pharmazeutischen Zusammensetzungen, in Pflanzenschutzformulierungen, in Wasch- oder Reinigungsmitteln oder in Farb- oder Anstrichmitteln statt. Die Pflanzenschutzmittel enthalten ein oder mehrere Pestizide.

Die kosmetischen, dermatologischen oder pharmazeutischen Zusammensetzungen, die Pflanzenschutzformulierungen, die Wasch- oder Reinigungsmittel oder die Farb- oder Anstrichmittel enthalten die eine oder die mehreren Verbindungen der Formel (I) vorzugsweise in Mengen von 0,01 bis 10,0 Gew.-%, besonders bevorzugt in Mengen von 0,1 bis 5,0 Gew.-% und insbesondere bevorzugt in Mengen von 0,2 bis 3,0 Gew.-%, jeweils bezogen auf das Gesamtgewicht der fertigen kosmetischen, dermatologischen oder pharmazeutischen Zusammensetzungen, Pflanzenschutzformulierungen, Wasch-oder Reinigungsmittel oder Farb- oder Anstrichmittel.

Die kosmetischen, dermatologischen oder pharmazeutischen Zusammensetzungen, die Pflanzenschutzformulierungen, die Wasch- oder Reinigungsmittel oder die Farb- oder Anstrichmittel haben Viskositäten vorzugsweise im Bereich von 50 bis 200 000 mPa · s, besonders bevorzugt im Bereich von 500 bis 100 000 mPa · s, insbesondere bevorzugt im Bereich von 2 000 bis 50 000 mPa · s und außerordentlich bevorzugt im Bereich von 5 000 bis 30 000 mPa · s (20 °C, Brookfield RVT, RV-Spindel-Satz; 20 Umdrehungen pro Minute).

Die kosmetischen, dermatologischen oder pharmazeutischen Zusammensetzungen liegen vorzugsweise in Form von Fluids, Gelen, Schäumen, Sprays, Lotions oder Cremes vor.

Die kosmetischen, dermatologischen oder pharmazeutischen Zusammensetzungen, die Pflanzenschutzformulierungen, die Wasch- oder Reinigungsmittel oder die Farb- oder Anstrichmittel sind vorzugsweise auf wässriger oder wässrig-alkoholischer Basis aufgebaut oder liegen als Emulsionen oder Dispersionen vor. Besonders bevorzugt liegen sie als Emulsionen vor und insbesondere bevorzugt liegen sie als Öl-in-Wasser Emulsionen vor.

Die kosmetischen, dermatologischen oder pharmazeutischen Zusammensetzungen, die Pflanzenschutzformulierungen, die Wasch- oder Reinigungsmittel oder die Farb- oder Anstrichmittel können als weitere Hilfs- und Zusatzstoffe alle für die jeweilige Anwendung üblicherweise verwendeten Substanzen enthalten, beispielsweise Öle, Wachse, Emulgatoren, Co-Emulgatoren, Dispergatoren, Tenside, Entschäumer, Solubilisatoren, Elektrolyte, Hydroxysäuren, Stabilisatoren, Polymere, Filmbildner, weitere (von den Verbindungen der Formel (I) verschiedene) Verdicker, Gelierungsmittel, Überfettungsmittel, Rückfetter, antimikrobielle Wirkstoffe, biogene Wirkstoffe, Adstringentien, Aktivstoffe, deodorierende Stoffe, Sonnenschutzfilter, Antioxidantien, Oxidantien, Feuchthaltemittel, Lösungsmittel, Farbmittel, Pigmente, Perlglanzmittel, Duftstoffe, Trübungsmittel und/oder Silikone.

Unter den kosmetischen, dermatologischen oder pharmazeutischen Zusammensetzungen, den Pflanzenschutzformulierungen, den Wasch- oder Reinigungsmitteln oder den Farb- oder Anstrichmitteln, die auf wässriger oder wässrig-alkoholischer Basis aufgebaut sind, sind diejenigen Zusammensetzungen bevorzugt, die ein oder mehrere Tenside enthalten.

Die kosmetischen, dermatologischen oder pharmazeutischen Zusammensetzungen, die Pflanzenschutzformulierungen, die Wasch- oder Reinigungsmittel oder die Farb- oder Anstrichmittel besitzen pH-Werte von vorzugsweise 2 bis 11, besonders bevorzugt von 4,5 bis 8,5 und insbesondere bevorzugt von 5,5 bis 6,5.

Die nachfolgenden Beispiele und Anwendungen sollen die Erfindung näher erläutern, ohne sie jedoch darauf zu beschränken. Bei allen Prozentangaben handelt es sich um Gewichts-% (Gew.-%), sofern nicht explizit anders angegeben.

Verdickungsleistungen wurden unter folgenden Bedingungen gemessen: Brookfield RVT; 20 °C; RV-Spindel-Satz; 20 Umdrehungen pro Minute)

Versuchsbeispiele:

A) Herstellung von Isosorbiddicaprylat

In einer 1 Liter-Rührapparatur mit Stickstoffüberleitung werden 219,0 g (1,5 mol) Isosorbid und 461,4 g (3,2 mol) Caprylsäure unter Rühren und Stickstoffüberleitung auf 180 °C erhitzt. Der Reaktionsansatz wird solange auf 180 °C erhitzt, bis kein Reaktionswasser mehr abdestilliert (etwa 28 h). Anschließend wird die Temperatur schrittweise bis auf 210 °C erhöht (insgesamt über etwa 30 h). Die Reaktion ist dann beendet, wenn eine Restsäurezahl von < 2 mg KOH /g erreicht ist. Es wird eine klare, rotbraune Flüssigkeit erhalten.

Weitere analytische Kennzahlen des Reaktionsproduktes:

| | |
|---|---|
| Säurezahl: | 0,8 mg KOH/g gemessen gemäß DIN EN ISO 2114 |
| Hydroxyl-Zahl: | 25,2 mg KOH/g gemessen in Anlehnung an DIN 53240-2 nach Methode OHZ-A |
| Verseifungszahl: | 54,6 mg KOH/g gemessen gemäß DIN EN ISO 3681 |

Zur weiteren Aufreinigung wurde das Produkt bei einem Druck von ≤ 1 mbar und einer Sumpftemperatur von 210 °C bis 240 °C destilliert. Es werden 251,6 g einer gelben klaren Flüssigkeit erhalten.

Das Isosorbiddicaprylat besitzt folgende Zusammensetzung:

| Substanz | Gew.-% |
|---|---|
| Isosorbidmonocaprylat | 9,4 |
| Isosorbidd icaprylat | 89,6 |

(fortgesetzt)

| Substanz | Gew.-% |
|---|---|
| Rest | 1 |

B) Bestimmung der Verdickungsleistung

Unter Verwendung von Genapol® LRO (Sodium Laureth-2 Sulfate, 27 Gew.-% in Wasser) und Genagen® KB (Cocobetain, 30 Gew.-% in Wasser) und zusätzlich Wasser wurde eine Mischung hergestellt, die die beiden Tenside im Gewichtsverhältnis 8 : 2 zu 15 Gew.-% in Wasser enthält. Zu dieser Mischung wurde das Isosorbiddicaprylat aus Herstellbeispiel A) zugegeben und die Viskosität der resultierenden Zusammensetzung bestimmt. Die Ergebnisse sind in der folgenden Tabelle 1 wiedergegeben.

Tabelle 1 gemessene Viskositäten

| Zugegebene Substanz; Menge [Gew.-%] | Viskosität [mPa · s] |
|---|---|
| Keine | 135 |
| Isosorbiddicaprylat [1 Gew.-%] | 2390 |

Wie den Ergebnissen der Tabelle 1 zu entnehmen ist, bewirkt Isosorbiddicaprylat eine signifikante Verdickung.

C) Anwendungsbeispiele

Die erfindungsgemäße Verwendung kann beispielsweise in folgenden Formulierungen stattfinden. Die Formulierungen werden unter Verwendung des Isosorbiddicaprylats aus Herstellbeispiel A) hergestellt.

Formulierungbeispiel 1: Sehr mildes EO- und Sulfat-freies Shampoo

| Phase | Inhaltsstoff | Gew.-% |
|---|---|---|
| A | Wasser | ad 100 |
| B | Sorbitol | 1,0 |
| C | Hostapon® SG<br>Sodium Cocoyl Glycinate<br>Genagen® KB<br>Coco-Betaine<br>Plantacare® 818 UP<br>Coco Glucoside<br>Isosorbiddicaprylat | 30,0<br><br>15,0<br><br>9,23<br><br>1,0 |
| D | Lactic Acid 25 Gew.-%ig in Wasser | 3,25 |
| E | Konservierungsmittel | q.s. |
| Herstellung:<br>I Gebe B zu A und rühre bis eine klare Lösung erhalten wird<br>II Gebe C zu I und rühre bis die Lösung homogen ist<br>III Stelle den pH-Wert mit D auf 7,0 - 7,2 ein<br>IV Gebe E zu III | | |

Formulierungsbeispiel 2: Milde Gesichtsreinigung

| Phase | Inhaltsstoff | Gew.-% |
|---|---|---|
| A | Wasser<br>Glycerin | ad 100<br>20,0 |

(fortgesetzt)

| Phase | Inhaltsstoff | Gew.-% |
|---|---|---|
| B | Hostapon® SG<br>Sodium Cocoyl Glycinate<br>Lauric Acid<br>Myristic Acid<br>Stearic Acid<br>Isosorbiddicaprylat | 34,0<br><br>1,0<br>0,25<br>0,25<br>1,0 |
| C | Lactic Acid<br>Wasser | 0,9<br>6,8 |
| D | Genagen® CAB<br>Cocamidopropyl Betaine | 10,0 |
| E | Konservierungsmittel<br>Duftstoff | q.s.<br>q.s. |
| Herstellung:<br>I Mische Phase A und erwärme auf 80 °C<br>II Gebe Komponenten von B nach und nach zu I und rühre weiter bei 80 °C<br>III Mische Phase C und gebe zu II<br>IV Lasse auf 60 °C abkühlen und gebe D zu III<br>V Bei 40 °C gebe E zu IV | | |

Formulierungsbeispiel 3: Handgeschirrspülmittel

| Inhaltsstoff | Gew. % |
|---|---|
| Hostapur® SAS 60 (Alkansulfonat, 60 Gew.-% in Wasser) | 40,0 |
| Hostapur® OS liquid (Sodium C14-16 Alkyl Sulfonate, 40 Gew.-% in Wasser) | 11,0 |
| Isosorbidd icaprylat | 1,0 |
| Genagen® CAB (Cocoamidopropylbetain, 30 Gew.-% in Wasser) | 3,0 |
| Konservierungsmittel | q.s. |
| Wasser | ad 100 |

Formulierungsbeispiel 4: Oberflächenreiniger (Allzweckreiniger)

| Inhaltsstoff | Gew.-% |
|---|---|
| Hostapur® SAS 60 (Alkansulfonat, 60 Gew.-% in Wasser) | 5,0 |
| Genapol® UD 080 (Undecanol + 8 EO) | 2,0 |
| Isosorbidd icaprylat | 1,0 |
| Konservierungsmittel | q.s. |
| Wasser | ad 100 |

Herstellung der Formulierungsbeispiele 3 und 4:

Die Hälfte der Wassermenge wird vorgelegt und die Komponenten werden in der Reihenfolge wie in den Tabellen aufgeführt eingerührt. Die restliche Menge Wasser wird dann nachgegeben. Es resultieren klare, wässrige Zusammensetzungen.

## Patentansprüche

1. Verwendung einer oder mehrerer Verbindungen der Formel (I)

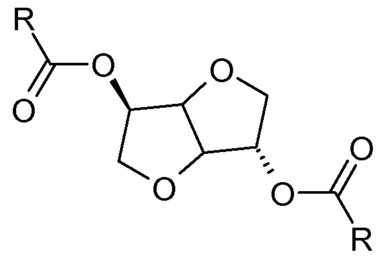

(I)

worin

R eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist,
wobei die eine oder die mehreren Verbindungen der Formel (I) in einer Zusammensetzung enthaltend eine oder mehrere andere Substanzen ausgewählt aus der Gruppe bestehend aus Sorbitol, Sorbitolestern, Sorbitan, Sorbitanestern, Isosorbid, Isosorbidmonoestern und Carbonsäuren verwendet werden
und die OH-Zahl (als mgKOH/g) der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbidmonoestern und Carbonsäuren in der Zusammensetzung kleiner oder gleich 250 ist, und wobei die Zusammensetzung eine oder mehrere Verbindungen ausgewählt aus Sorbitol und Sorbitolestern enthält,
und wobei die Zusammensetzung eine oder mehrere Verbindungen der Formel (I) und zusätzlich
II) 0,001 bis 0,2 Gewichtsteile an dem einem oder den mehreren Isosorbidmonoestern der Formel (II)

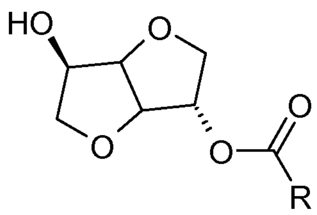

(II)

worin R die oben angegebene Bedeutung besitzt,
enthält, jeweils bezogen auf 1,0 Gewichtsteile an der einen oder den mehreren Verbindungen der Formel (I),

als Verdicker.

2. Verwendung nach Anspruch 1, **dadurch gekennzeichnet, dass** der Rest R in Formel (I) ein linearer gesättigter Alkylrest mit 7 bis 9 Kohlenstoffatomen ist.

3. Verwendung nach Anspruch 2, **dadurch gekennzeichnet, dass** der Rest R in Formel (I) ein linearer gesättigter Alkylrest mit 7 Kohlenstoffatomen ist.

4. Verwendung nach einem oder mehreren der Ansprüche 1 bis 3, **dadurch gekennzeichnet, dass** die Menge der einen oder der mehreren Verbindungen der Formel (I), bezogen auf die Gesamtmenge der Verbindungen ausgewählt aus der Gruppe bestehend aus der einen oder den mehreren Verbindungen der Formel (I), Sorbitol, Sorbitolestern, Sorbitan, Sorbitanestern, Isosorbid, Isosorbidmonoestern und Carbonsäuren größer oder gleich 70 Gew.-%, besonders bevorzugt größer oder gleich 80 Gew.-% und insbesondere bevorzugt größer oder gleich 85 Gew.-% ist.

5. Verwendung nach einem oder mehreren der Ansprüche 1 bis 4, **dadurch gekennzeichnet, dass** der Isosorbidmonoester Isosorbidmonocaprylat ist.

6. Verwendung nach einem oder mehreren der Ansprüche 1 bis 5, **dadurch gekennzeichnet, dass** die Zusammensetzung

II) 0,01 bis 0,15 und besonders bevorzugt 0,05 bis 0,13 Gewichtsteile an dem einem oder den mehreren Isosorbidmonoestern der Formel (II), wobei der Isosorbidmonoester vorzugsweise Isosorbidmonocaprylat ist,

enthält, jeweils bezogen auf 1,0 Gewichtsteile an der einen oder den mehreren Verbindungen der Formel (I) und vorzugsweise bezogen auf 1,0 Gewichtsteile an Isosorbidd icaprylat.

7. Verwendung nach einem oder mehreren der Ansprüche 1 bis 6, **dadurch gekennzeichnet, dass** die Zusammensetzung eine oder mehrere Verbindungen der Formel (I) und einen oder mehrere Sorbitanester aus Sorbitan und Carbonsäuren $R^aCOOH$, vorzugsweise ausgewählt aus Sorbitanestern aus 1,4- und/oder 1,5-Sorbitan und Carbonsäuren $R^aCOOH$, wobei $R^a$ eine lineare oder verzweigte, gesättigte Alkylgruppe mit 5 bis 11, vorzugsweise 7 bis 9 und besonders bevorzugt 7 Kohlenstoffatomen oder eine lineare oder verzweigte, ein- oder mehrfach ungesättigte Alkenylgruppe mit 5 bis 11, vorzugsweise 7 bis 9 und besonders bevorzugt 7 Kohlenstoffatomen ist, enthält, und das Gewichtsverhältnis der einen oder der mehreren Verbindungen der Formel (I) zu dem einen oder den mehreren soeben genannten Sorbitanestern von 70 : 30 bis 100 : 0, vorzugsweise von 80 : 20 bis 100 : 0, besonders bevorzugt von 90 : 10 bis 100 : 0 und insbesondere bevorzugt von 95 : 5 bis 100 : 0 ist.

8. Verwendung nach Anspruch 7, **dadurch gekennzeichnet, dass** der eine oder die mehreren Sorbitanester aus Sorbitan und Carbonsäuren $R^aCOOH$ ausgewählt sind aus Sorbitanestern aus Sorbitan und Caprylsäure und vorzugsweise ausgewählt sind aus Sorbitanestern aus 1,4- und/oder 1,5- Sorbitan und Caprylsäure und der Sorbitanester besonders bevorzugt Sorbitandicaprylat ist.

9. Verwendung nach einem oder mehreren der Ansprüche 1 bis 8, **dadurch gekennzeichnet, dass** die OH-Zahl der Mischung aus der einen oder den mehreren Verbindungen der Formel (I) und der einen oder den mehreren Verbindungen ausgewählt aus der Gruppe bestehend aus Sorbitol, Sorbitolestern, Sorbitan, Sorbitanestern, Isosorbid, Isosorbidmonoestern und Carbonsäuren in der Zusammensetzung kleiner oder gleich 200, besonders bevorzugt kleiner oder gleich 150 und insbesondere bevorzugt kleiner oder gleich 100 ist.

10. Verwendung nach einem oder mehreren der Ansprüche 1 bis 9, **dadurch gekennzeichnet, dass** die Zusammensetzung die eine oder die mehreren Verbindungen der Formel (I) in Mengen von mindestens 50 Gew.-%, vorzugsweise in Mengen von mindestens 60 Gew.-% und besonders bevorzugt in Mengen von mindestens 70 Gew.-% enthält, jeweils bezogen auf das Gesamtgewicht der fertigen Zusammensetzung.

11. Verwendung nach einem oder mehreren der Ansprüche 1 bis 10 in kosmetischen, dermatologischen oder pharmazeutischen Zusammensetzungen, in Pflanzenschutzformulierungen, in Wasch- oder Reinigungsmitteln oder in Farb- oder Anstrichmitteln.

12. Verwendung nach Anspruch 11, **dadurch gekennzeichnet, dass** die kosmetischen, dermatologischen oder pharmazeutischen Zusammensetzungen, die Pflanzenschutzformulierungen, die Wasch- oder Reinigungsmittel oder die Farb- oder Anstrichmittel die eine oder die mehreren Verbindungen der Formel (I) in Mengen von 0,01 bis 10,0 Gew.-%, vorzugsweise in Mengen von 0,1 bis 5,0 Gew.-% und besonders bevorzugt in Mengen von 0,2 bis 3,0 Gew.-% enthalten, jeweils bezogen auf das Gesamtgewicht der fertigen kosmetischen, dermatologischen oder pharmazeutischen Zusammensetzungen, Pflanzenschutzformulierungen, Wasch- oder Reinigungsmittel oder Farb- oder Anstrichmittel.

13. Verwendung nach Anspruch 11 oder 12, **dadurch gekennzeichnet, dass** die kosmetischen, dermatologischen oder pharmazeutischen Zusammensetzungen, die Pflanzenschutzformulierungen, die Wasch- oder Reinigungsmittel oder die Farb- oder Anstrichmittel auf wässriger oder wässrig-alkoholischer Basis aufgebaut sind oder als Emulsion oder Dispersion vorliegen und vorzugsweise als Emulsion vorliegen.

14. Verwendung nach einem oder mehreren der Ansprüche 11 bis 13, **dadurch gekennzeichnet, dass** die kosmetischen, dermatologischen oder pharmazeutischen Zusammensetzungen, die Pflanzenschutzformulierungen, die Wasch- oder Reinigungsmittel oder die Farb- oder Anstrichmittel einen pH-Wert von 2 bis 11, vorzugsweise von 4,5 bis 8,5 und besonders bevorzugt von 5,5 bis 6,5 besitzen.

**Claims**

1. Use of one or more compounds of the formula (I)

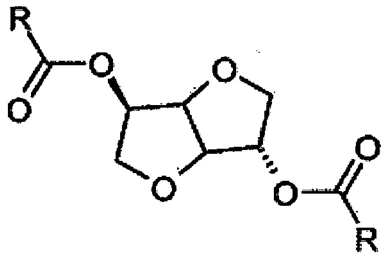

(I)

in which

R is a straight-chain or branched saturated alkyl group having 5 to 11, preferably 7 to 9 and particularly preferably 7 carbon atoms or a straight-chain or branched mono- or polyunsaturated alkenyl group having 5 to 11, preferably 7 to 9 and particularly preferably 7 carbon atoms,
wherein the one or more compounds of the formula (I) are used in a composition comprising one or more other substances selected from the group consisting of sorbitol, sorbitol esters, sorbitan, sorbitan esters, isosorbide, isosorbide monoesters and carboxylic acids,
and the hydroxyl value (as mg KOH/g) of the mixture of the one or more compounds of the formula (I) and the one or more compounds selected from the group consisting of sorbitol, sorbitol esters, sorbitan, sorbitan esters, isosorbide, isosorbide monoesters and carboxylic acids in the composition is smaller than or equal to 250, and wherein the composition comprises one or more compounds selected from sorbitol and sorbitol esters,
and wherein the composition comprises one or more compounds of the formula (I) and additionally II) from 0.001 to 0.2 part by weight of the one or more isosorbide monoesters of the formula (II)

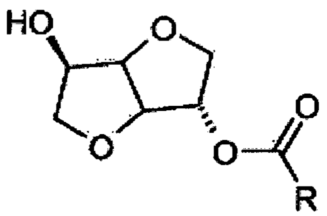

(II)

where R has the meaning given above,
in each case based on 1.0 part by weight of the one or more compounds of the formula (I),

as thickeners.

2. Use according to Claim 1, **characterized in that** the radical R in formula (I) is a straight-chain saturated alkyl radical having 7 to 9 carbon atoms.

3. Use according to Claim 2, **characterized in that** the radical R in formula (I) is a straight-chain saturated alkyl radical having 7 carbon atoms.

4. Use according to one or more of Claims 1 to 3, **characterized in that** the amount of the one or more compounds of the formula (I), based on the total amount of the compounds selected from the group consisting of the one or more compounds of the formula (I), sorbitol, sorbitol esters, sorbitan, sorbitan esters, isosorbide, isosorbide monoesters and carboxylic acids is greater than or equal to 70% by weight, particularly preferably greater than or equal to 80% by weight and especially preferably greater than or equal to 85%.

5. Use according to one or more of Claims 1 to 4, **characterized in that** the isosorbide monoester is isosorbide monocaprylate.

**6.** Use according to one or more of Claims 1 to 5, **characterized in that** the composition comprises

II) from 0.01 to 0.15 and particularly preferably from 0.05 to 0.13 part by weight of the one or more isosorbide monoesters of the formula (II), where the isosorbide monoester is preferably isosorbide monocaprylate,

in each case based on 1.0 part by weight of the one or more compounds of the formula (I) and preferably based on 1.0 part by weight of isosorbide dicaprylate.

**7.** Use according to one or more of Claims 1 to 6, **characterized in that** the composition comprises one or more compounds of the formula (I) and one or more sorbitan esters of sorbitan and carboxylic acids $R^aCOOH$, preferably selected from sorbitan esters of 1,4- and/or 1,5-sorbitan and carboxylic acids $R^aCOOH$, where $R^a$ is a straight-chain or branched saturated alkyl group having 5 to 11, preferably 7 to 9 and particularly preferably 7 carbon atoms or a straight-chain or branched mono- or polyunsaturated alkenyl group having 5 to 11, preferably 7 to 9 and particularly preferably 7 carbon atoms, and the weight ratio of the one or more compounds of the formula (I) to the one or more sorbitan esters just mentioned is from 70:30 to 100:0, preferably from 80:20 to 100:0, particularly preferably from 90:10 to 100:0 and especially preferably from 95:5 to 100:0.

**8.** Use according to Claim 7, **characterized in that** the one or more sorbitan esters of sorbitan and carboxylic acids $R^aCOOH$ are selected from sorbitan esters of sorbitan and caprylic acid and are preferably selected from sorbitan esterns of 1,4- and/or 1,5-sorbitan and caprylic acid and the sorbitan ester is particularly preferably sorbitan dicaprylate.

**9.** Use according to one or more of Claims 1 to 8, **characterized in that** the hydroxyl value of the mixture of the one or more compounds of the formula (I) and the one or more compounds selected from the group consisting of sorbitol, sorbitol esters, sorbitan, sorbitan esters, isosorbide, isosorbide monoesters and carboxylic acids in the composition is smaller than or equal to 200, particularly preferably smaller than or equal to 150 and especially preferably smaller than or equal to 100.

**10.** Use according to one or more of Claims 1 to 9, **characterized in that** the composition comprises the one or more compounds of the formula (I) in amounts of at least 50% by weight, preferably in amounts of at least 60% by weight and particularly preferably in amounts of at least 70% by weight, in each case based on the total weight of the finished composition.

**11.** Use according to one or more of Claims 1 to 10 in cosmetic, dermatological or pharmaceutical compositions, in crop protection formulations, in washing or cleaning compositions or in paints or coatings.

**12.** Use according to Claim 11, **characterized in that** the cosmetic, dermatological or pharmaceutical compositions, the crop protection formulations, the washing or cleaning compositions or the paints or coatings comprise the one or more compounds of the formula (I) in amounts of from 0.01 to 10.0% by weight, preferably in amounts of from 0.1 to 5.0% by weight and particularly preferably in amounts of from 0.2 to 3.0% by weight, in each case based on the total weight of the finished cosmetic, dermatological or pharmaceutical compositions, crop protection formulations, washing or cleaning compositions or paints or coatings.

**13.** Use according to Claim 11 or 12, **characterized in that** the cosmetic, dermatological or pharmaceutical compositions, the crop protection formulations, the washing or cleaning compositions or the paints or coatings are formulated on an aqueous or aqueous-alcoholic basis or are present as emulsion or dispersion and preferably as emulsion.

**14.** Use according to one or more of Claims 11 to 13, **characterized in that** the cosmetic, dermatological or pharmaceutical compositions, the crop protection formulations, the washing or cleaning compositions or the paints or coatings have a pH of from 2 to 11, preferably from 4.5 to 8.5 and particularly preferably from 5.5 to 6.5.

## Revendications

**1.** Utilisation d’un ou de plusieurs composés de formule (I)

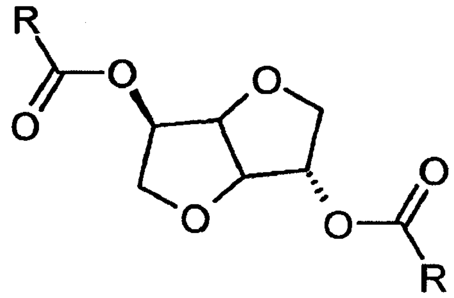

(I)

dans laquelle

R représente un groupe alkyle saturé linéaire ou ramifié de 5 à 11, de préférence 7 à 9 et de manière particulièrement préférée 7 atomes de carbone, ou un groupe alcényle mono- ou polyinsaturé, linéaire ou ramifié, de 5 à 11, de préférence 7 à 9, et de manière particulièrement préférée 7 atomes de carbone,
le ou les composés de formule (I) étant utilisés dans une composition contenant une ou plusieurs autres substances choisies dans le groupe constitué par le sorbitol, les esters de sorbitol, le sorbitane, les esters de sorbitane, l'isosorbide, les monoesters d'isosorbide et les acides carboxyliques,
et l'indice OH (en mg KOH/g) du mélange du ou des composés de formule (I) et du ou des composés choisis dans le groupe constitué par le sorbitol, les esters de sorbitol, le sorbitane, les esters de sorbitane, l'isosorbide, les monoesters d'isosorbide et les acides carboxyliques dans la composition étant inférieur ou égal à 250,
et la composition contenant un ou plusieurs composés choisis parmi le sorbitol et les esters de sorbitol,
et la composition contenant un ou plusieurs composés de formule (I) et en outre

II) 0,001 à 0,2 partie en poids du ou des monoesters d'isosorbide de formule (II)

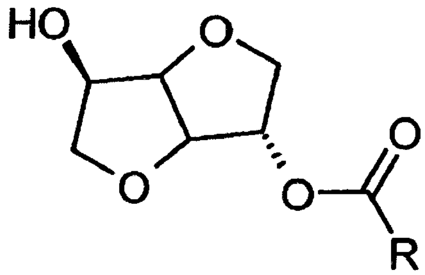

(II)

dans laquelle R a la signification indiquée précédemment, à chaque fois par rapport à 1,0 partie en poids du ou des composés de formule (I),
en tant qu'épaississant.

2. Utilisation selon la revendication 1, **caractérisée en ce que** le radical R dans la formule (I) est un radical alkyle saturé linéaire de 7 à 9 atomes de carbone.

3. Utilisation selon la revendication 2, **caractérisée en ce que** le radical R dans la formule (I) est un radical alkyle saturé linéaire de 7 atomes de carbone.

4. Utilisation selon une ou plusieurs des revendications 1 à 3, **caractérisée en ce que** la quantité du ou des composés de formule (I), par rapport à la quantité totale des composés choisis dans le groupe constitué par le ou les composés de formule (I), le sorbitol, les esters de sorbitol, le sorbitane, les esters de sorbitane, l'isosorbide, les monoesters d'isosorbide et les acides carboxyliques, est supérieure ou égale à 70 % en poids, de manière particulièrement préférée supérieure ou égale à 80 % en poids, et de manière notamment préférée supérieure ou égale à 85 % en poids.

5. Utilisation selon une ou plusieurs des revendications 1 à 4, **caractérisée en ce que** le monoester d'isosorbide est

le monocaprylate d'isosorbide.

**6.** Utilisation selon une ou plusieurs des revendications 1 à 5, **caractérisée en ce que** la composition contient

II) 0,01 à 0,15 et de manière particulièrement préférée 0,05 à 0,13 partie en poids du ou des monoesters d'isosorbide de formule (II), le monoester d'isosorbide étant de préférence le monocaprylate d'isosorbide,

à chaque fois par rapport à 1,0 partie en poids du ou des composés de formule (I) et de préférence par rapport à 1,0 partie en poids de dicaprylate d'isosorbide.

**7.** Utilisation selon une ou plusieurs des revendications 1 à 6, **caractérisée en ce que** la composition contient un ou plusieurs composés de formule (I) et un ou plusieurs esters de sorbitane constitués de sorbitane et d'acides carboxyliques $R^aCOOH$, de préférence choisis parmi les esters de sorbitane constitués de 1,4-et/ou 1,5-sorbitane et d'acides carboxyliques $R^aCOOH$, $R^a$ étant un groupe alkyle saturé linéaire ou ramifié de 5 à 11, de préférence 7 à 9 et de manière particulièrement préférée 7 atomes de carbone, ou un groupe alcényle mono-ou polyinsaturé, linéaire ou ramifié, de 5 à 11, de préférence 7 à 9 et de manière particulièrement préférée 7 atomes de carbone, et le rapport en poids entre le ou les composés de formule (I) et ledit ou lesdits esters de sorbitane est de 70:30 à 100:0, de préférence de 80:20 à 100:0, de manière particulièrement préférée de 90:10 à 100:0 et de manière notamment préférée de 95:5 à 100:0.

**8.** Utilisation selon la revendication 7, **caractérisée en ce que** le ou les esters de sorbitane constitués de sorbitane et d'acides carboxyliques $R^aCOOH$ sont choisis parmi les esters de sorbitane constitués de sorbitane et d'acide caprylique, et sont de préférence choisis parmi les esters de sorbitane constitués de 1,4- et/ou 1,5-sorbitane et d'acide caprylique, et l'ester de sorbitane est de manière particulièrement préférée le dicaprylate de sorbitane.

**9.** Utilisation selon une ou plusieurs des revendications 1 à 8, **caractérisée en ce que** l'indice OH du mélange du ou des composés de formule (I) et du ou des composés choisis dans le groupe constitué par le sorbitol, les esters de sorbitol, le sorbitane, les esters de sorbitane, l'isosorbide, les monoesters d'isosorbide et les acides carboxyliques dans la composition est inférieur ou égal à 200, de manière particulièrement préférée inférieur ou égal à 150, et de manière notamment préférée inférieur ou égal à 100.

**10.** Utilisation selon une ou plusieurs des revendications 1 à 9, **caractérisée en ce que** la composition contient le ou les composés de formule (I) en quantités d'au moins 50 % en poids, de préférence en quantités d'au moins 60 % en poids et de manière particulièrement préférée en quantités d'au moins 70 % en poids, à chaque fois par rapport au poids total de la composition finie.

**11.** Utilisation selon une ou plusieurs des revendications 1 à 10 dans des compositions cosmétiques, dermatologiques ou pharmaceutiques, dans des formulations phytosanitaires, dans des détergents ou dans des colorants ou des peintures.

**12.** Utilisation selon la revendication 11, **caractérisée en ce que** les compositions cosmétiques, dermatologiques ou pharmaceutiques, les formulations phytosanitaires, les détergents ou les colorants ou les peintures contiennent le ou les composés de formule (I) en quantités de 0,01 à 10,0 % en poids, de préférence en quantités de 0,1 à 5,0 % en poids et de manière particulièrement préférée en quantités de 0,2 à 3,0 % en poids, à chaque fois par rapport au poids total des compositions cosmétiques, dermatologiques ou pharmaceutiques, des formulations phytosanitaires, des détergents ou des colorants ou des peintures finis.

**13.** Utilisation selon la revendication 11 ou 12, **caractérisée en ce que** les compositions cosmétiques, dermatologiques ou pharmaceutiques, les formulations phytosanitaires, les détergents ou les colorants ou les peintures sont à base aqueuse ou aqueuse-alcoolique, ou se présentent sous la forme d'une émulsion ou d'une dispersion, et se présentent de préférence sous la forme d'une émulsion.

**14.** Utilisation selon une ou plusieurs des revendications 11 à 13, **caractérisée en ce que** les compositions cosmétiques, dermatologiques ou pharmaceutiques, les formulations phytosanitaires, les détergents ou les colorants ou les peintures présentent un pH de 2 à 11, de préférence de 4,5 à 8,5 et de manière particulièrement préférée de 5,5 à 6,5.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010108738 A2
- EP 2239315 A